# EUROPEAN PATENT APPLICATION

(11) **EP 4 814 927 A1**
(43) Date of publication of application: **30.09.2026**
(21) Application number: 25165659.1
(22) Date of filing: 24.03.2025
(51) Int. Cl.: G09B 9/00, A61N 1/39, G09B 23/28

(54) **EXTERNAL DEFIBRILLATOR WITH AUGMENTED/VIRTUAL REALITY TRAINING**

(71) Applicant: Everest Acquisition Entity, LLC, Wilmington, DE 19805 (US)
(72) Inventor: UPADHYAY, Hemant, 5656 AG Eindhoven (NL); RAYKWAR, Sangita, 5656 AG Eindhoven (NL)
(74) Representative: Weilnau, Carsten

(57) **Abstract**

An external rescue/training defibrillator (40) (e.g., an automatic external rescue/training defibrillator (40) or a semi-automatic external rescue/training defibrillator (40)) operable between a rescue operation mode for executing a defibrillation treatment of a cardiac emergency and a training operation mode for simulating the defibrillation treatment of the cardiac emergency. When the external rescue/training defibrillator (40) is set in the rescue operation mode, the external rescue/training defibrillator (40) is operable to control an execution of the defibrillation treatment of the cardiac emergency including a conditional shock delivery. When the external rescue/training defibrillator (40) is set in the training operation mode, the external rescue/training defibrillator (40) is operable to control a digital simulation of the defibrillation treatment of the cardiac emergency by an augmented defibrillator trainer (60) or a virtual defibrillator trainer (70). The digital simulation of the defibrillation treatment of the cardiac emergency can include a digital simulation of a cardiopulmonary resuscitation.

## Description

### FIELD OF THE INVENTION

The present disclosure generally relates to external rescue/training defibrillators, particularly automatic external rescue/training defibrillators and semi-automatic external rescue/training defibrillators. The present disclosure particularly relates to incorporating augmented reality defibrillation treatment training and virtual reality defibrillation treatment training into external rescue/training defibrillators.

### BACKGROUND OF THE INVENTION

Automated external rescue/training defibrillators and semi-automatic external rescue/training defibrillators (collectively herein "AEDs") are critical devices used in medical emergencies to analyze and treat cardiac emergencies. AED training devices (e.g., HeartStart Onsite Trainer and HeartStart FRx Trainer) are tools designed for teaching and practicing the use by responders of the AEDs (e.g., HeartStart Onsite AED and HeartStart Home AED). As known in the art of the present disclosure, these training devices simulate the look and functionality of actual AEDs, but are specifically designed for training purposes and are not configured to deliver an actual defibrillation shock. More particularly, the training devices are equipped with reusable, low-adhesive training pads for adults and/or children, depending on the model and setup, and often come with a range of simulated cardiac emergency scenarios whereby the training devices provides audio instructions to be followed by responders, laypeople and professionals, during execution of the simulated cardiac emergency scenario(s).

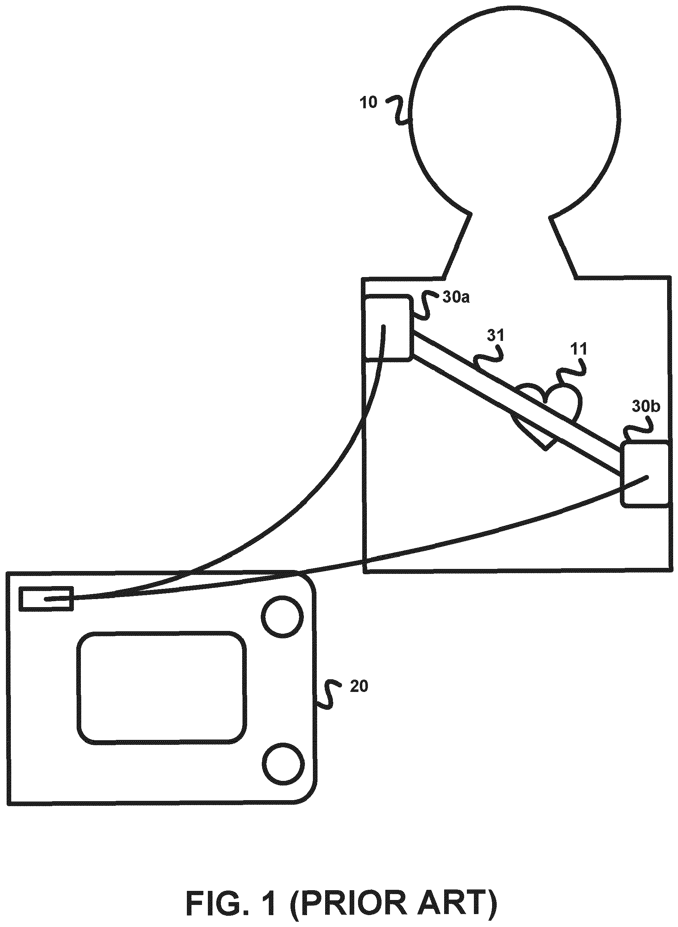

FIG. 1 illustrates an exemplary training device 20 equipped with training pads 30a and 30b aligned along a guide 31 over a heart location 11 of a CPR manikin 10. In operation, training device 20 provides audio instructions, optionally including coaching in performing cardiopulmonary resuscitation, for simulating the look and functionality of an AED (not shown) via various simulated cardiac emergency scenarios including responder actions related to simulated improper pad placement by training device 20, responder actions related to simulated shock decision(s) by training device 20and/or responder actions related to simulated no-shook decision(s) by training device 20.

While training devices for AEDs have proven to be beneficial, such benefits have been limited by a lack of realistic feedback and a limited breath of training scenarios. Additionally, maintenance and upkeep, technology updates and specificity to AED model are additional limiting factors of the benefits of training devices for AEDs.

### SUMMARY OF THE INVENTION

For purpose of addressing the aforementioned drawbacks with conventional training devices for AEDs, the present disclosure provides an incorporation of control by external defibrillator of an augmented defibrillator trainer or a virtual defibrillator trainer to facilitate a digital simulation of a defibrillation treatment for a cardiac emergency.

For purposes of describing and claiming the present disclosure, the term "augmented defibrillator trainer" broadly encompasses any device/tool configured in accordance with the present disclosure to augment a real-world view of a training environment for a trainee of an external rescue/training defibrillator, whereby the device/tool is controllable by the external rescue/training defibrillator to overlay digital elements representative of a simulated defibrillation treatment of a cardiac emergency into the real-word view of the training environment by the trainee.

For purposes of describing and claiming the present disclosure, the term "visual defibrillator trainer" broadly encompasses any device/tool configured in accordance with the present disclosure to generate a virtual view of a training environment for a trainee of an external rescue/training defibrillator, whereby the device/tool is controllable by the external rescue/training defibrillator to generate digital representation of an interaction of the trainee within the virtual view of the training environment during a simulated defibrillation treatment of a cardiac emergency by the external rescue/training defibrillator.

The present disclosure can be embodied as (1) an external defibrillation rescue/training system for selectively executing or simulating a defibrillation treatment of a cardiac emergency, (2) an external rescue/training defibrillator for controlling a selective execution or simulation of a defibrillation treatment of a cardiac emergency, (3) a defibrillation rescue/training controller for controlling a selective execution or simulation of a defibrillation treatment of a cardiac emergency, and (4) an external defibrillation rescue/training method executable by an external rescue/training defibrillator controlling a selective execution or simulation of a defibrillation treatment of a cardiac emergency.

Various embodiments of an external defibrillation rescue/training system of the present disclosure employ an external rescue/training defibrillator and further employ an augmented defibrillator trainer and/or a virtual defibrillator trainer.

The external rescue/training defibrillator is operable between a rescue operation mode for executing a defibrillation treatment for a cardiac emergency and a training operation mode for simulating a defibrillation treatment for the cardiac emergency.

When the external rescue/training defibrillator is set in the rescue mode, the external rescue/training defibrillator is configured to execute the defibrillation treatment for the cardiac emergency including a conditional shock delivery.

When the external rescue/training defibrillator is set in the training operation mode and when the external rescue/training defibrillator is in electrical communication with the augmented defibrillator trainer (wired or wireless), the external rescue/training defibrillator is configured to control a digital simulation of the defibrillation treatment of the cardiac emergency by the augmented defibrillator trainer.

When the external rescue/training defibrillator is set in the training operation mode and when the external rescue/training defibrillator is in electrical communication with the virtual defibrillator trainer (wired or wireless), the external rescue/training defibrillator is configured to control a digital simulation of the defibrillation treatment of the cardiac emergency by the virtual defibrillator trainer.

Various embodiments of an external rescue/training defibrillator of the present disclosure employ a defibrillation rescue/training controller and a defibrillation shock module.

The defibrillation rescue/training controller is operable to set the external rescue/training defibrillator in a rescue operation mode for executing a defibrillation treatment for a cardiac emergency or in a training operation mode for simulating the defibrillation treatment for the cardiac emergency.

When the external rescue/training defibrillator is set in the rescue operation mode, the defibrillation rescue/training controller is configured to control an execution of the defibrillation treatment for the cardiac emergency including a conditional shock delivery by the shock defibrillation module.

When the external rescue/training defibrillator is set in the training operation mode and when the external rescue/training defibrillator is in electrical communication with an augmented defibrillator trainer (wired or wireless), the defibrillation rescue/training controller is configured to control a digital simulation of the defibrillation treatment of the cardiac emergency by the augmented defibrillator trainer.

When the external rescue/training defibrillator is set in the training operation mode and when the external rescue/training defibrillator is in electrical communication with a virtual defibrillator trainer (wired or wireless), the defibrillation rescue/training controller is configured to control a digital simulation of the defibrillation treatment of the cardiac emergency by the virtual defibrillator trainer.

Various embodiments of defibrillation rescue/training controller of the present disclosure employ a non-transitory machine-readable storage medium encoded with instructions for execution by one or more processors.

The non-transitory machine-readable storage medium includes the instructions to set an external rescue/training defibrillator in a rescue operation mode for executing a defibrillation treatment for a cardiac emergency or in a training operation mode for simulating the defibrillation treatment for the cardiac emergency.

When the external rescue/training defibrillator is set in the rescue operation mode, the non-transitory machine-readable storage medium further includes instructions to control an execution of the defibrillation treatment for the cardiac emergency by an external rescue/training defibrillator including a conditional shock delivery by external rescue/training defibrillator.

When the external rescue/training defibrillator is set in the training operation mode and when the defibrillation rescue/training controller is in electrical communication with an augmented defibrillator trainer (wired or wireless), the non-transitory machine-readable storage medium further includes instructions control a digital simulation of the defibrillation treatment of the cardiac emergency by the augmented defibrillator trainer.

When the external rescue/training defibrillator is set in the training operation mode and when the defibrillation rescue/training controller is in electrical communication with a virtual defibrillator trainer (wired or wireless), the non-transitory machine-readable storage medium further includes instructions control a digital simulation of the defibrillation treatment of the cardiac emergency by the virtual defibrillator trainer.

Various embodiments of an external defibrillation rescue/training method of the present disclosure are executable by an external rescue/training defibrillator operable between a rescue operation mode for executing a defibrillation treatment for a cardiac emergency and a training operation mode for simulating the defibrillation treatment for the cardiac emergency.

When the external rescue/training defibrillator is set in the rescue operation mode, the external rescue/training defibrillator executes the defibrillation treatment for the cardiac emergency including a conditional shock delivery by external rescue/training defibrillator.

When the external rescue/training defibrillator is set in the training operation mode and when the external rescue/training defibrillator is in electrical communication with an augmented defibrillator trainer (wired or wireless), the external rescue/training defibrillator controls a digital simulation of the defibrillation treatment of the cardiac emergency by the augmented defibrillator trainer.

When the external rescue/training defibrillator is set in the training operation mode and when the external rescue/training defibrillator is in electrical communication with a virtual defibrillator trainer (wired or wireless), the external rescue/training defibrillator controls a digital simulation of the defibrillation treatment of the cardiac emergency by the virtual defibrillator trainer.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates an exemplary embodiment of an external defibrillation training device as known in the art of the present disclosure;
FIG. 2 illustrates an exemplary embodiment of various components of an external defibrillation rescue/training system in accordance with the present disclosure.
FIG. 3A illustrates a flowchart representative of an exemplary embodiment of an external defibrillation rescue/training method in accordance with the present disclosure;
FIG. 3B illustrates a flowchart representative of an exemplary embodiment of a training scenario processing method in accordance with the present disclosure;
FIGS. 4A-4H illustrate exemplary embodiments of digital elements in accordance with the present disclosure;
FIGS. 5A-5H illustrate exemplary augmented reality scenes during an augmented digital simulation of a defibrillation treatment of a cardiac emergency in accordance with the present disclosure;
FIGS. 6A-6H illustrate exemplary virtual reality scenes during an augmented digital simulation of a defibrillation treatment of a cardiac emergency in accordance with the present disclosure; and
FIG. 7 illustrates an exemplary embodiment of a defibrillation rescue/training controller in accordance with the present disclosure.

### DETAILED DESCRIPTION OF THE PREFERRED EMBODIMENTS

The present disclosure is directed to incorporating augmented reality and virtual reality into training responders, laypersons and professionals, in the use of external rescue/training defibrillators.

More particularly, the incorporation of an augmented defibrillator trainer into an external rescue/training defibrillator in accordance with the present disclosure for cardiac emergency training purposes provides for the overlay of digital information into a physical environment enacted with an actual person or CPR manikin. The augmented defibrillator trainer of the present disclosure can graphically display step-by-step instructions and animations overlaid onto the physical world to guide a trainee through the process of deploying an external rescue/training defibrillator and performing CPR techniques.

Furthermore, the incorporation of a virtual defibrillator trainer into an external rescue/training defibrillator in accordance with the present disclosure for cardiac emergency training purposes provides a creation of a virtual realistic environment whereby a trainee can experience simulated emergency situations. The virtual defibrillator trainer of the present disclosure can graphically display step-by-step instructions and animations within the virtual world to guide a trainee through the process of deploying an external rescue/training defibrillator and performing CPR techniques.

Both an augmented defibrillator trainer and a virtual defibrillator trainer are operated in accordance with the present disclosure to expose a trainee to a wide variety of cardiac emergency scenarios involving different patient parameters (e.g., age, gender, and medical background) and/or different locations (e.g., an office, a public venue or a home). Additionally, Both an augmented defibrillator trainer and a virtual defibrillator trainer are operated in accordance with the present disclosure to provide interactive learning optionally with haptic feedback devices and with immediate feedback and post-training assessments.

For purposes of describing and claiming the present disclosure, terms of the art of the present disclosure, but not limited to, "defibrillation", "defibrillator", "defibrillating shock", "electrocardiogram (ECG)", "controller" and "module" are to be interpreted as known in the art of the present disclosure and as exemplary described in the present disclosure.

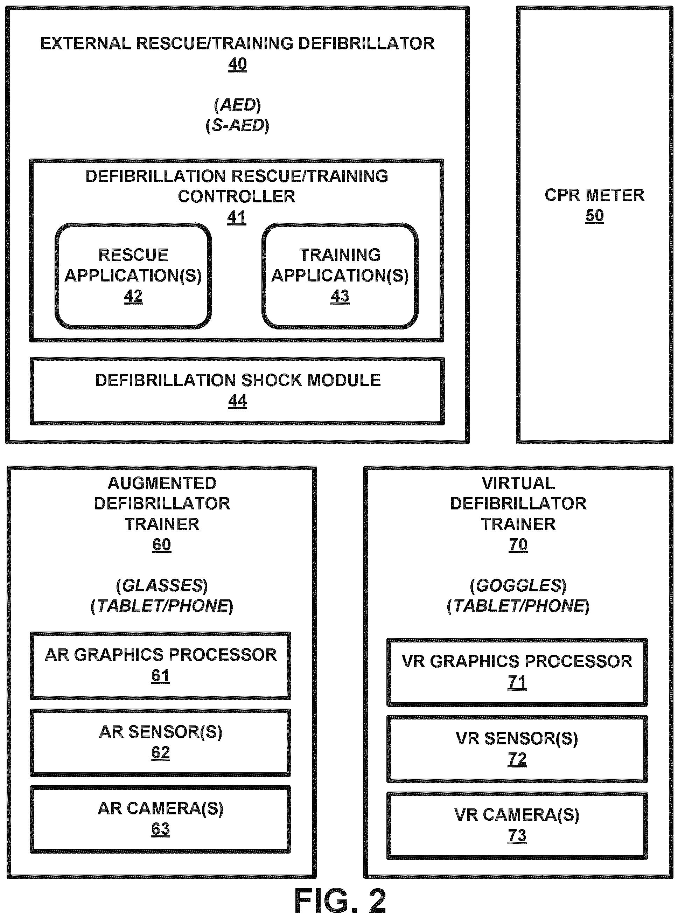

To facilitate an understanding of the present disclosure, the following description of FIG. 2 describes and teaches exemplary embodiments of systems and devices and in accordance with the present disclosure. From the description of FIG. 2, those having ordinary skill in the art of the present disclosure will appreciate how to apply the present disclosure to make and use additional embodiments of systems and devices in accordance with the present disclosure.

FIG. 2 illustrates devices of an external defibrillation rescue/training system of the present disclosure.

A first device of an external defibrillation rescue/training system of the present disclosure is an external rescue/training defibrillator 40.

In one exemplary embodiment, external rescue/training defibrillator 10 is an automatic external rescue/training defibrillator (AED), as known in the art of the present disclosure or herein conceived, incorporating the inventive principles of the present disclosure as further described herein.

In another exemplary embodiment, external rescue/training defibrillator 10 is a semi-automatic external rescue/training defibrillator (S-AED), as known in the art of the present disclosure or herein conceived, incorporating the inventive principles of the present disclosure as further described herein.

In practice of the present disclosure, external rescue/training defibrillator 40 is operable between a rescue operation mode for executing a defibrillation treatment for a cardiac emergency and a training operation mode for simulating a defibrillation treatment for the cardiac emergency. To this end, external rescue/training defibrillator 40 employs a defibrillation rescue/training controller 41 and a defibrillation shock module 44.

Defibrillation rescue/training controller 41 encompasses structural configurations, as understood in the art of the present disclosure, of an application specific main board or an application specific integrated circuit for executing rescue application(s) 42 when the external rescue/training defibrillator 40 is operating in the rescue operation mode and further configured to execute training application(s) 43 when the external rescue/training defibrillator 40 is operating in the training operation mode. The structural configuration of defibrillation rescue/training controller 41 can include, but is not limited to, processor(s), computer-usable/computer readable storage medium(s), an operating system, peripheral device controller(s), slot(s) and port(s).

Still referring to FIG. 2, rescue application(s) 42 encompass modules incorporated within defibrillation rescue/training controller 41 that can be configured as an electronic circuit (e.g., electronic components and/or hardware) and/or an executable program (e.g., executable software stored on non-transitory computer readable medium(s) and/or firmware) for implementing shock advisory methods and shock delivery methods as known in the art of the present disclosure or hereinafter conceived.

In one exemplary shock advisory embodiment, rescue application(s) 42 implement a C-shock advisory broadly encompassing all methods, as known in the art of the present disclosure or hereinafter conceived, for analyzing and rhythm classifying an ECG of a heart of a patient including artifacts as known in the art of the present disclosure resulting from an administration of chest compressions to the heart of the patient (i.e., a corrupt ECG).

An execution of C-shock advisory renders either (1) a shock decision based upon a determinate classification of the corrupt ECG as having a shockable cardiac rhythm (e.g., a ventricular fibrillation (VF) rhythm or a ventricular tachycardia (VT) rhythm), (2) a non-shock decision based upon a determinate classification of the corrupt ECG as having a non-shockable cardiac rhythm (e.g., a pulseless electrical activity rhythm or an asystole rhythm, (3) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm), or (4) a terminate rescue decision based upon a determinate classification of the corrupt ECG as having a sustained cardiac rhythm (e.g., a return of an organized cardiac rhythm with or without a sustained return of a spontaneous circulation).

A non-limiting example of a C-shock advisory is an Arrythmia Recognition Technology (ART) as known in the art of the present disclosure.

In practice, the C-shock advisory, the undecided shock decision and/or the terminate rescue decision can be omitted and/or additional shock advisory decision(s) can be derived from a corrupt ECG.

In a second exemplary shock advisory embodiment, rescue application(s) 42 implement a F-shock advisory broadly encompassing all methods, as known in the art of the present disclosure or hereinafter conceived, for analyzing and rhythm classifying an ECG of a heart of a patient excluding artifacts as known in the art of the present resulting from a termination/suspension of an administration of chest compressions to the heart of the patient (i.e., a clean ECG).

An execution of F-shock advisory renders either (1) a shock decision based upon a determinate classification of the clean ECG as having a shockable cardiac rhythm (e.g., a ventricular fibrillation (VF) rhythm or a ventricular tachycardia (VT) rhythm), (2) a non-shock based upon a determinate classification of the clean ECG as having a non-shockable cardiac rhythm (e.g., a pulseless electrical activity rhythm or an asystole rhythm), (3) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm), or (4) a terminate rescue decision based upon a determinate classification of the clean ECG as having a sustained cardiac rhythm (e.g., a return of an organized cardiac rhythm with or without a sustained return of spontaneous circulation).

A non-limiting example a F-shock advisory is a Patient Analysis System (PAS) as known in the art of the present disclosure.

In practice, the F-shock advisory, the undecided shock decision and/or the terminate rescue decision can be omitted and/or additional shock advisory decision(s) can be derived from a clean ECG.

Still referring to FIG. 2, defibrillating shock module 44 is structurally configured as known in the art of the present disclosure to deliver an electric therapy to a heart of a patient as controlled by defibrillation rescue/training controller 41.

In one exemplary embodiment, defibrillating shock module 13 employs a high voltage capacitor bank (not shown) for storing a high voltage via a high voltage charger and a power supply. Defibrillating shock module 13 further employs a switching/isolation circuit (not shown) for selectively applying a specific waveform of an electric energy charge from the high voltage capacitor bank to electrode pads/paddles attached to the patient as controlled by defibrillation rescue/training controller 41. In practice, the defibrillating shock may have any waveform as known in the art of the present disclosure. A non-limiting example of such a waveform is a biphasic truncated waveform.

In one exemplary shock delivery embodiment, rescue application(s) 42 implement a C-shock delivery broadly encompassing methods, as known in the art of the present disclosure or hereinafter conceived, for delivering a defibrillating shock energy from defibrillating shock module 44 to a heart of a patient corresponding to a shock decision derived by the C-shock advisory in accordance with rules/guidelines associated with a rescue protocol (e.g., American Heart Association rules/guidelines).

In a second exemplary shock delivery embodiment, rescue application(s) 42 implement a F-shock delivery broadly encompassing methods, as known in the art of the present disclosure or hereinafter conceived, for delivering a defibrillating shock from defibrillating shock module 44 to a heart of a patient corresponding to a shock decision derived by the F-shock advisory in accordance with rules/guidelines associated with a rescue protocol (e.g., American Heart Association rules/guidelines).

Still referring to FIG. 2, an optional second device of an external defibrillation rescue/training system of the present disclosure is a cardiopulmonary resuscitation (CPR) meter 50 as known in the art of the present disclosure or hereinafter conceived.

In one exemplary embodiment, CPR meter 50 is a tool, utilized for measuring CPR parameters as a responder applies CPR to a patient via CPR meter 50. Non-limiting examples of the CPR parameters include compression rate, ventilation rate, compression rate, chest recoil and interruptions/hands off time.

Still referring to FIG 2, a third device of an external defibrillation rescue/training system of the present disclosure is an augmented defibrillator trainer 60 configured in accordance with the present disclosure to augment a real-world view of a training environment for a trainee of external rescue/training defibrillator 40, whereby augmented defibrillator trainer 60 is controllable by the external rescue/training defibrillator 40 to overlay digital elements representative of a simulated defibrillation treatment of a cardiac emergency into the real-word view of the training environment by the trainee. Non-limiting examples of augmented defibrillator trainer 60 are an augmented reality glasses and augmented reality tablet/phone configured in accordance with the present disclosure.

In practice of the present disclosure, augmented defibrillator trainer 60 employs augmented reality graphics processor 61, as known in the art of the present disclosure or hereinafter conceived, that is configured in accordance with the present disclosure to process instructions/commands from defibrillation rescue/training controller 41 for overlaying digital elements representative of a simulated defibrillation treatment of a cardiac emergency into the real-word view of the training environment by the trainee as will be further described in the present disclosure.

Further in practice of the present disclosure, augmented defibrillator trainer 60 further employs augmented reality sensor(s) 62 and augmented reality camera(s) 63, as known in the art of the present disclosure or hereinafter conceived, for respectively communicating sensing data and images to defibrillation rescue/training controller 41, whereby defibrillation rescue/training controller 41 implements hand recognition/tracking techniques as known in the art of the present disclosure or hereinafter conceived.

The following is a description of several exemplary augmented training scenarios executable by defibrillation rescue/training controller 41 in conjunction with augmented defibrillator trainer 60.

An exemplary augmented pad placement scenario involves defibrillation rescue/training controller 41 controlling a simulation of a pad placement on an object (e.g., person or manikin) within an augmented physical environment provided by augmented defibrillation trainer 60 with status/feedback icon(s) and/or instructions related to proper pad placement within the augmented physical environment.

Subsequent to proper pad placement, an exemplary augmented single shock/single conversion scenario involves the defibrillation rescue/training controller 41 controlling a simulation of a detection of a shockable rhythm and shock delivery within an augmented physical environment provided by augmented defibrillation trainer 60, followed by a simulation of a post-shock detection of a non-shockable rhythm within the augmented physical environment. This augmented single shock/single conversion scenario can (1) further involve iconic status and/or instructions within the augmented physical environment related to automatic defibrillation shock delivery or semi-automatic defibrillation shock delivery and/or (2) further involve iconic status and/or instructions related to pre-shock delivery CPR actions and/or post-shock delivery CPR actions.

Subsequent to proper pad placement, an exemplary augmented multiple shocks/single conversion scenario involves the defibrillation rescue/training controller 41 sequentially controlling multiple simulations of a detection of a shockable rhythm and shock delivery within an augmented physical environment provided by augmented defibrillation trainer 60, followed by a simulation of a post-shock detection of a non-shockable rhythm within the augmented physical environment. Each simulation of a detection of a shockable rhythm and shock delivery can involve a single shock delivery per shock detection or a series of shock deliveries during per shock detection. This augmented multiple shocks/single conversion scenario can (1) further involve iconic status and/or instructions within the augmented physical environment related to automatic defibrillation shock deliveries or semi-automatic defibrillation shock deliveries and/or (2) further involve iconic status and/or instructions related to pre-shock delivery CPR actions and/or post-shock delivery CPR actions.

Subsequent to proper pad placement, an exemplary augmented multiple shocks/multiple conversions scenario involves the defibrillation rescue/training controller 41 controlling a first simulations of a detection of a shockable rhythm and shock delivery within an augmented physical environment provided by augmented defibrillation trainer 60, followed by a first simulation of a post-shock detection of a non-shockable rhythm within the augmented physical environment, followed by a second detection of a shockable rhythm and shock delivery within the augmented physical environment and followed by a second simulation of a post-shock detection of a non-shockable rhythm within the augmented physical environment. Each simulation of a detection of a shockable rhythm and shock delivery can involve a single shock delivery per shock detection or a series of shock deliveries during per shock detection. This augmented multiple shocks/multiple conversions scenario can (1) further involve iconic status and/or instructions within the augmented physical environment related to automatic defibrillation shock deliveries or semi-automatic defibrillation shock deliveries, and/or (2) further involve iconic status and/or instructions related to pre-shock delivery CPR actions and/or post-shock delivery CPR actions.

Subsequent to proper pad placement, an exemplary augmented no shock scenario involves the defibrillation rescue/training controller 41 controlling a simulation of a detection of a non-shockable rhythm within an augmented physical environment provided by augmented defibrillation trainer 60. This augmented non-shock scenario further involve iconic status and/or instructions related to pre-no shock delivery CPR actions and/or post-no shock delivery CPR actions.

Still referring to FIG 2, a fourth device of an external defibrillation rescue/training system of the present disclosure is a virtual defibrillator trainer 70 configured in accordance with the present disclosure to any device/tool configured in accordance with the present disclosure to generate a virtual view of a training environment for a trainee of external rescue/training defibrillator 40, whereby the device/tool is controllable by external rescue/training defibrillator 40 to generate a digital representation of an interaction of the trainee within the virtual view of the training environment during a simulated defibrillation treatment of a cardiac emergency by external rescue/training defibrillator 40. Non-limiting examples of virtual defibrillator trainer 70 are virtual reality goggles and virtual reality tablet/phone configured in accordance with the present disclosure.

In practice of the present disclosure, virtual defibrillator trainer 70 employs a virtual reality graphics processor 71, as known in the art of the present disclosure or hereinafter conceived, that is configured in accordance with the present disclosure to process instructions/commands from defibrillation rescue/training controller 41 for generating a digital representation of an interaction of the trainee within the virtual view of the training environment during a simulated defibrillation treatment of a cardiac emergency by external rescue/training defibrillator 40as will be further described in the present disclosure.

Further in practice of the present disclosure, virtual defibrillator trainer 70 further employs virtual reality sensor(s) 72 and virtual reality camera(s) 73, as known in the art of the present disclosure or hereinafter conceived, for respectively communicating sensing data and images to defibrillation rescue/training controller 41, whereby defibrillation rescue/training controller 41 implements hand recognition/tracking techniques as known in the art of the present disclosure or hereinafter conceived.

The following is a description of several exemplary virtual training scenarios executable by defibrillation rescue/training controller 41 in conjunction with virtual defibrillator trainer 70.

An exemplary virtual pad placement scenario involves the defibrillation rescue/training controller 41 controlling a simulation of a pad placement on an object (e.g., person or manikin) within an interactive virtual environment provided by virtual defibrillation trainer 70 with status/feedback icon(s) and/or instructions related to proper pad placement within the interactive virtual environment.

Subsequent to proper pad placement, an exemplary virtual single shock/single conversion scenario involves the defibrillation rescue/training controller 41 controlling a simulation of a detection of a shockable rhythm and shock delivery within an interactive virtual environment provided by virtual defibrillation trainer 70, followed by a simulation of a post-shock detection of a non-shockable rhythm within the interactive virtual environment. This virtual single shock/single conversion scenario can (1) further involve iconic status and/or instructions within the interactive virtual environment related to automatic defibrillation shock delivery or semi-automatic defibrillation shock delivery and/or (2) further involve iconic status and/or instructions related to pre-shock delivery CPR actions and/or post-shock delivery CPR actions.

Subsequent to proper pad placement, an exemplary virtual multiple shocks/single conversion scenario involves the defibrillation rescue/training controller 41 sequentially controlling multiple simulations of a detection of a shockable rhythm and shock delivery within an interactive virtual environment provided by virtual defibrillation trainer 70, followed by a simulation of a post-shock detection of a non-shockable rhythm within the interactive virtual environment. Each simulation of a detection of a shockable rhythm and shock delivery can involve a single shock delivery per shock detection or a series of shock deliveries during per shock detection. This virtual multiple shocks/single conversion scenario can (1) further involve iconic status and/or instructions within the interactive virtual environment related to automatic defibrillation shock deliveries or semi-automatic defibrillation shock deliveries and/or (2) further involve iconic status and/or instructions related to pre-shock delivery CPR actions and/or post-shock delivery CPR actions.

Subsequent to proper pad placement, an exemplary virtual multiple shocks/multiple conversions scenario involves the defibrillation rescue/training controller 41 controlling a first simulations of a detection of a shockable rhythm and shock delivery within an interactive virtual environment provided by virtual defibrillation trainer 70, followed by a first simulation of a post-shock detection of a non-shockable rhythm within the interactive virtual environment, followed by a second detection of a shockable rhythm and shock delivery within the interactive virtual environment and followed by a second simulation of a post-shock detection of a non-shockable rhythm within the interactive virtual environment. Each simulation of a detection of a shockable rhythm and shock delivery can involve a single shock delivery per shock detection or a series of shock deliveries during per shock detection. This virtual multiple shocks/multiple conversions scenario can (1) further involve iconic status and/or instructions within the interactive virtual environment related to automatic defibrillation shock deliveries or semi-automatic defibrillation shock deliveries, and/or (2) further involve iconic status and/or instructions related to pre-shock delivery CPR actions and/or post-shock delivery CPR actions.

Subsequent to proper pad placement, an exemplary virtual no shock scenario involves the defibrillation rescue/training controller 41 controlling a simulation of a detection of a non-shockable rhythm within an interactive virtual environment provided by virtual defibrillation trainer 70. This virtual non-shock scenario further involves iconic status and/or instructions related to pre-no shock delivery CPR actions and/or post-no shock delivery CPR actions.

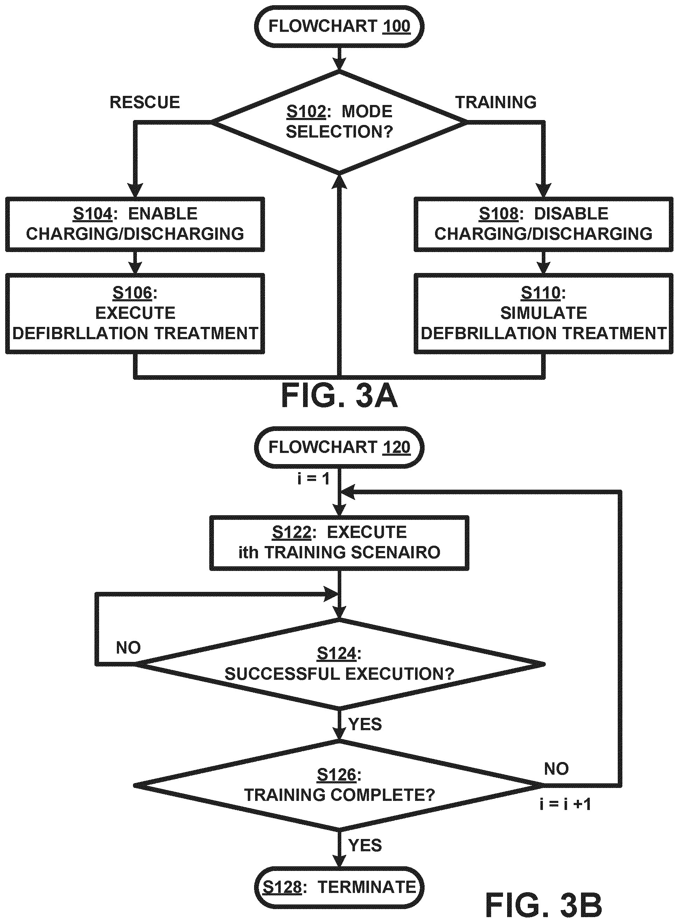

To facilitate a further understanding of the present disclosure, the following description of FIGS. 3A-6G describes and teaches exemplary embodiments of methods in accordance with the present disclosure. From the description of FIG. 2, those having ordinary skill in the art of the present disclosure will appreciate how to apply the present disclosure to make and use additional embodiments of methods in accordance with the present disclosure.

Referring to FIG. 3A, a flowchart 100 is representative of an exemplary embodiment of a defibrillation rescue/training method of the present disclosure. A stage S102 of flowchart 100 encompasses a responder setting of an external rescue/training defibrillator of the present disclosure (e.g., external rescue/training defibrillator 40 shown in FIG. 2 of the present disclosure) between a rescues operation mode for executing a defibrillation treatment for a cardiac emergency and a training operation mode for simulating a defibrillation treatment for the cardiac emergency.

If the external rescue/training defibrillator is set in the rescue mode during stage S102, then flowchart 100 sequentially proceeds to a stage S104 encompassing the external rescue/training defibrillator enabling a charging and a discharging of a defibrillation shock module (e.g., defibrillation shock module 44 shown in FIG. 2 of the present disclosure) and a stage S106 encompassing a controller of external rescue/training defibrillator (e.g., defibrillation rescue/training controller 41 as shown in FIG. 2 of the present disclosure) executing a defibrillation treatment for a cardiac emergency.

In one exemplary embodiment of stage S106, the controller of the external rescue/training defibrillator executes rescue applications of the present disclosure (e.g., rescue applications 42 as shown in FIG. 2 of the present disclosure. For example, the controller of the external rescue/training defibrillator can execute a C-shock advisory and a C-shock delivery for a rescue involving CPR. By further example the controller of the external rescue/training defibrillator can execute a F-shock advisory and a F-shock delivery for a rescue not involving CPR.

Still referring to FIG. 3A, if the external rescue/training defibrillator is set in the training mode during stage S102, then flowchart 100 sequentially proceeds to a stage S108 encompassing the external rescue/training defibrillator disabling a charging and a discharging of the defibrillation shock module and a stage S110 encompassing the controller of external rescue/training defibrillator simulating a defibrillation treatment for a cardiac emergency.

In one exemplary embodiment of stage S110, the controller of the external rescue/training defibrillator executes augmented training applications of the present disclosure (e.g., augmented versions of training applications 43 as shown in FIG. 2 of the present disclosure).

For example, when the training mode of the external rescue/training defibrillator involves an augmented defibrillator trainer of the present disclosure (e.g., augmented defibrillator trainer 60 as shown in FIG. 2 of the present disclosure, the controller of the external rescue/training defibrillator can execute the scenario based training applications including, but not limited to (1) an augmented pad placement scenario, (2) an augmented single shock/single conversion scenario, (3) an augmented multiple shocks/single conversion scenario, (4) an augmented multiple shocks/multiple conversions scenario and (5) an augmented no-shock scenario.

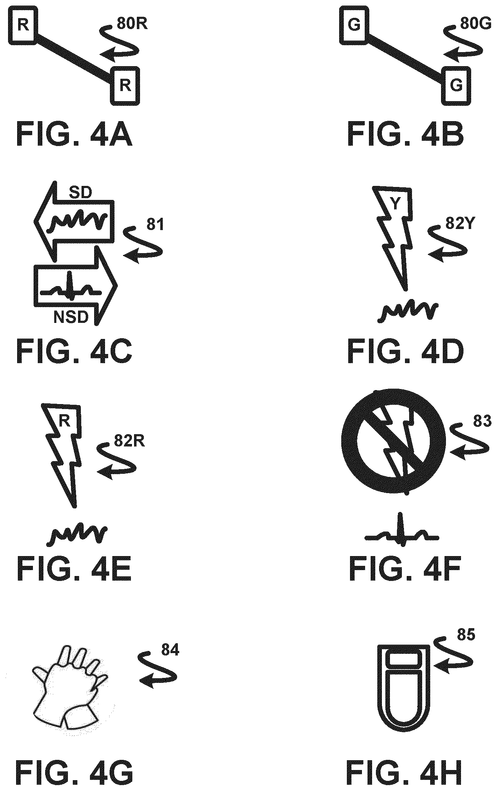

To this end, FIGS. 4A-4H illustrate exemplary status/feedback icons of the present disclosure that can be utilized in the augmented scenarios.

FIG. 4A illustrates an improper pad placement icon 80R that is highlighted in red (R).

FIG. 4B illustrates a proper pad placement icon 80G that is highlighted in green (G).

FIG. 4C illustrates a shock advisory execution in progress icon 81 which may result in a shock decision (SD) or a no-shock decision (NSD).

FIG. 4D illustrates, upon a shock decision, a charging pre-shock delivery icon 82Y that is highlighted in yellow (Y) as a caution that shock delivery is imminent.

FIG. 4E illustrates, upon a complete charging action, a discharging shock delivery icon 82R that is highlighted in red (R) as a warning the shock delivery is in progress.

FIG. 4F illustrates a no-shock decision icon 83.

FIG. 4G illustrates a recommended CPR icon 84 for training without a CPR meter.

FIG. 4H illustrates a recommended CPR icon 85 for training with a CPR meter.

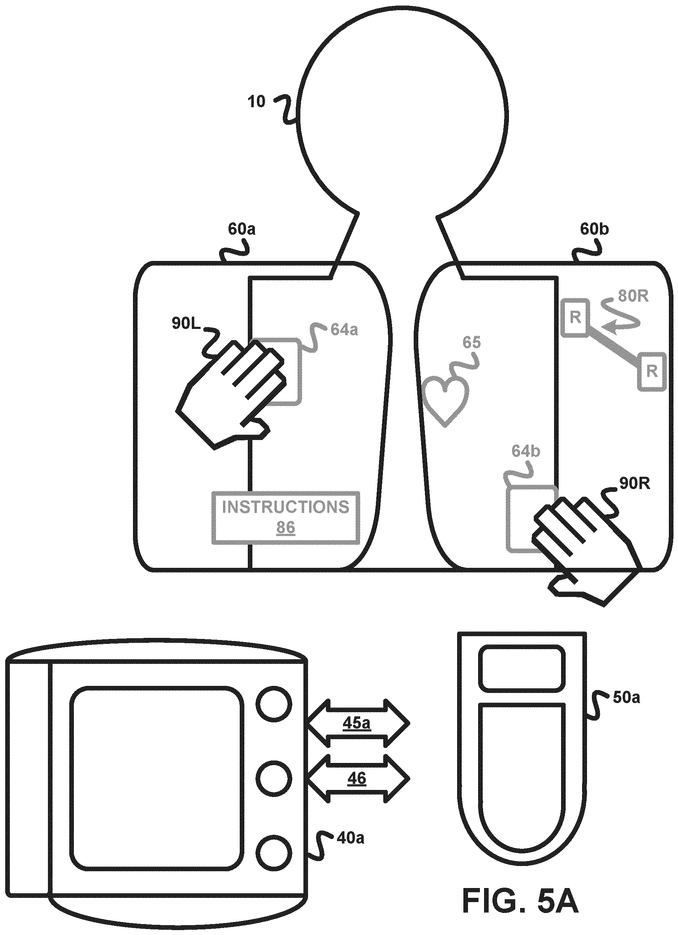

The following description of FIGS. 5A-5H is directed to an exemplary utilization of the status/feedback icon of the present disclosure (e.g., status/feedback icons shown in FIGS. 4A-4H) via exemplary augmented lens 60a and 60b of an augmented defibrillator trainer of the present during an execution of a previously described augmented training scenarios of the present disclosure.

FIGS. 5A-5H further illustrate an exemplary embodiment 40a of external rescue/training defibrillator 40 (FIG. 2 of the present disclosure) having a wireless/wired communication 45a with the augmented defibrillator trainer, and a wireless/wired communication 46 with an exemplary embodiment 50a of CPR meter 50 (FIG. 2 of the present disclosure. While not shown for clarity of the exemplary operation of augmented lens 60a and 60b, a defibrillation rescue/training controller of external rescue/training defibrillator 40a inputs sensing data and/or images from the augmented sensor(s)/camera(s) of the augmented defibrillator trainer to thereby command an augmented graphical processor of the augmented defibrillator trainer to generate the status/feedback icons and instructions as needed during an augmented training scenario.

For a simulated improper pad placement during an augmented pad placement scenario, FIG. 5A illustrates a real-world view through augmented lens 60a and 60b of a CPR manikin 10 and trainee hands 90L and 90R. As trainee hands 90L and 90R are being tracked by the augmented defibrillator trainer, FIG. 5A further illustrates a screen shot via augmented lens 60a and 60b of augmented defibrillation electrode pads 64a and 64b relative to an augmented heart 65, and of a simulated improper pad placement icon 80R informing the trainee hands 90L and 90R have not been properly placed in an anterior-posterior position. Additionally, an instruction box 86 can be provided with instructions for properly placing the electrode pads and/or external rescue/training defibrillator 40a can provide audio instructions for properly placing the electrode pads.

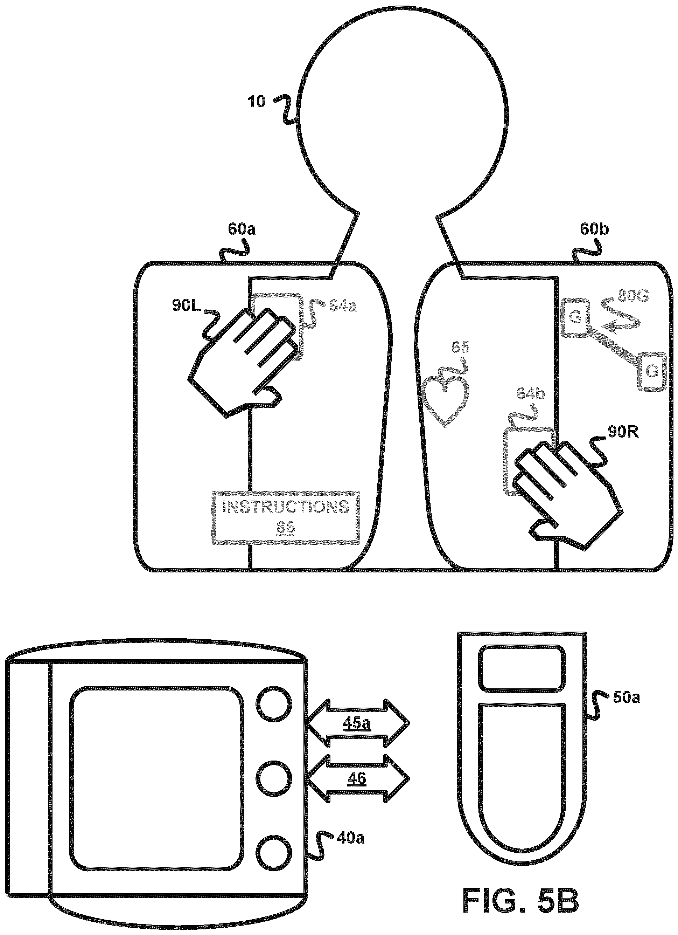

For a simulated proper pad placement during an augmented pad placement scenario, FIG. 5B illustrates a real-world view through augmented lens 60a and 60b of a CPR manikin 10 and trainee hands 90L and 90R. As trainee hands 90L and 90R are being tracked by the augmented defibrillator trainer, FIG. 5B further illustrates a screen shot via augmented lens 60a and 60b of augmented defibrillation electrode pads 64a and 64b relative to augmented heart 65, and of a simulated proper pad placement icon 80G informing the trainee hands 90L and 90R have been properly placed in an anterior-posterior position. Additionally, an instruction box 86 can be provided with a notification of properly placed electrode pads and/or external rescue/training defibrillator 40a can provide an audio notification s for properly placement of the electrode pads.

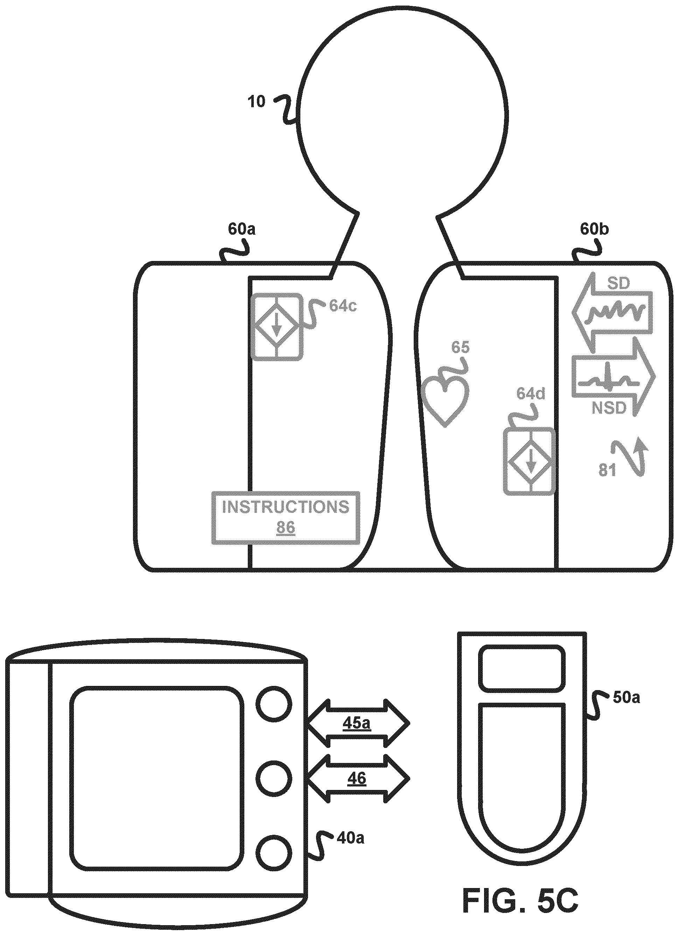

For a simulated shock advisory execution in progress without CPR, FIG. 5C illustrates a real-world view through augmented lens 60a and 60b of a CPR manikin 10 without trainee hands 90L and 90R. FIG. 5C further illustrates a screen shoot via augmented lens 60a and 60b of augmented defibrillation electrode pads 64c and 64d highlighting a sensing of electrical cardiac signals of augmented heart 65, and a shock advisory execution in progress icon 81. Additionally, instruction box 86 can be provided to inform the trainee of the progress of the shock advisory execution and/or external rescue/training defibrillator 40a can provide an audio notification of the progress of the shock advisory execution.

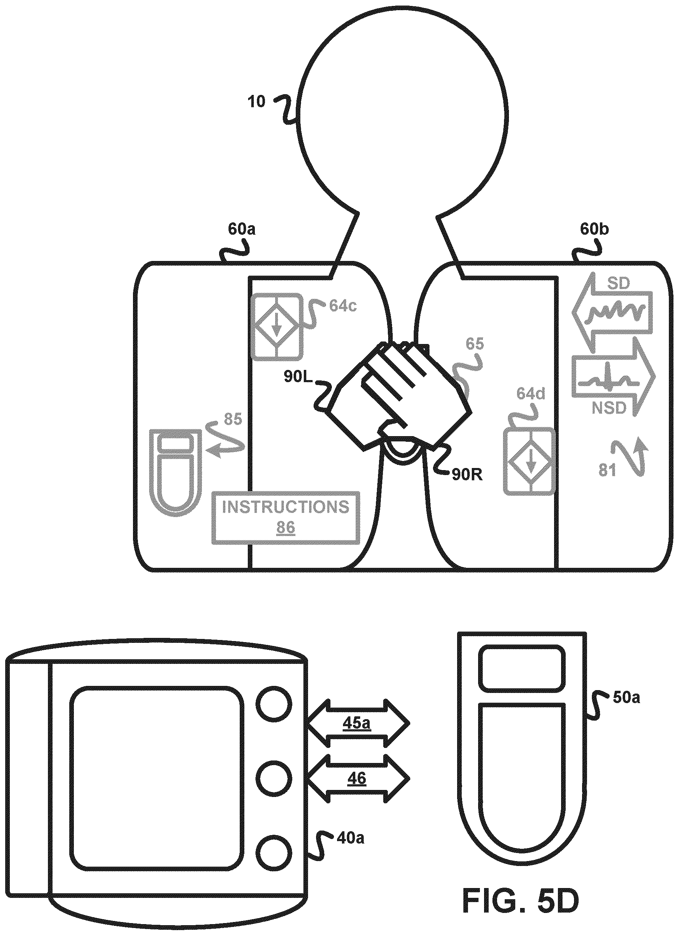

For a simulated shock advisory execution in progress with CPR, FIG. 5D illustrates a real-world view through augmented lens 60a and 60b of a CPR manikin 10 with trainee hands 90L and 90R performing CPR via CPR meter 50a. FIG. 5D further illustrates a screen shoot via augmented lens 60a and 60b of augmented defibrillation electrode pads 64c and 64d highlighting a sensing of electrical cardiac signals of augmented heart 65, and of shock advisory execution in progress icon 81. Additionally, instruction box 86 can be provided to inform the trainee of the progress of the shock advisory execution as well as CPR instructions and/or external rescue/training defibrillator 40a can provide an audio notification of the progress of the shock advisory execution as well as CPR instructions.

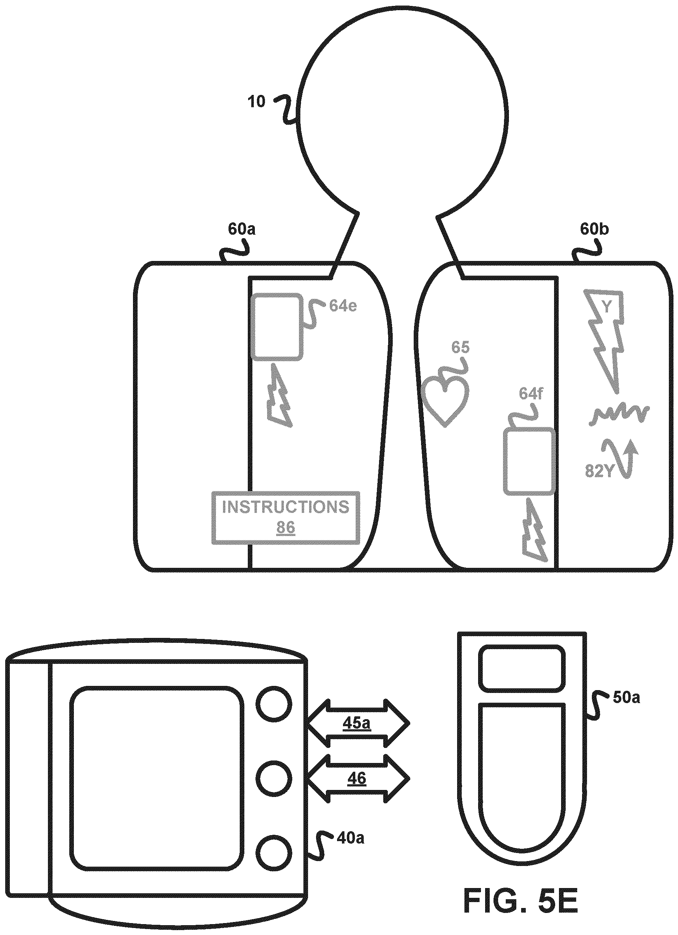

For a simulated shock decision/charging pre-shock delivery, FIG. 5E illustrates a real-world view through augmented lens 60a and 60b of a CPR manikin 10 without trainee hands 90L and 90R. FIG. 5E further illustrates a screen shoot via augmented lens 60a and 60b of augmented defibrillation electrode pads 64e and 64f highlighting a simulated charging pre-shock delivery and of charging pre-shock delivery icon 82Y. Additionally, instruction box 86 can be provided to inform the trainee details of the charging pre-shock delivery and/or external rescue/training defibrillator 40a can provide an audio notification of the charging pre-shock delivery. Also, external rescue/training defibrillator 40a can activate a shock charging button to simulate the charging pre-shock delivery and activate a shock delivery button upon completion of the full charging pre-shock delivery to simulate a semi-automatic version of external rescue/training defibrillator.

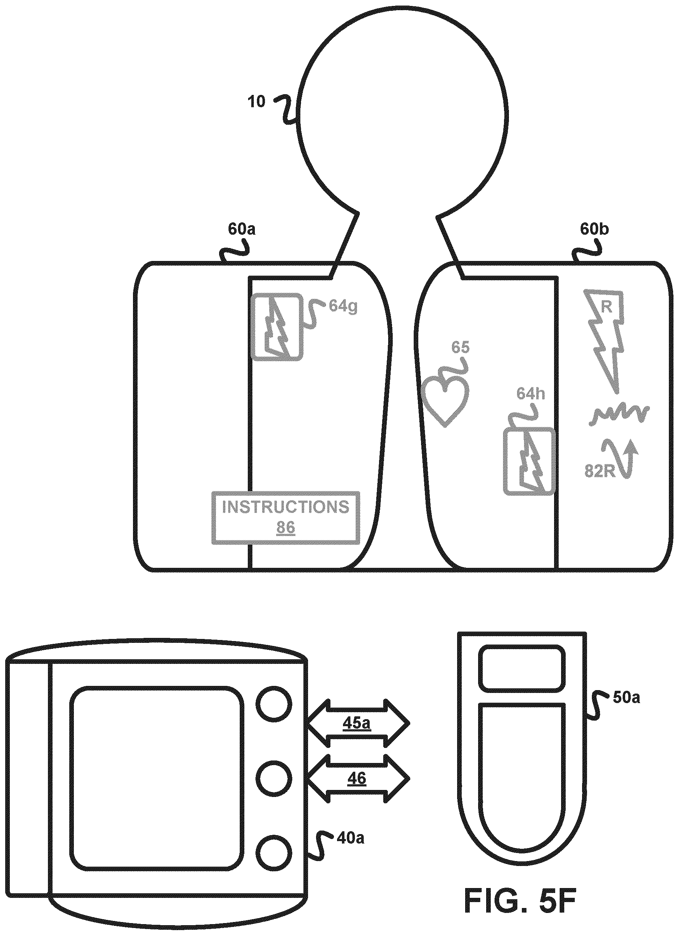

For a simulated shock decision/shock delivery, FIG. 5F illustrates a real-world view through augmented lens 60a and 60b of a CPR manikin 10 without trainee hands 90L and 90R. FIG. 5F further illustrates a screen shoot via augmented lens 60a and 60b of augmented defibrillation electrode pads 64g and 64h highlighting a shock delivery and of a shock delivery icon 82R. Additionally, instruction box 86 can be provided to inform the trainee details of the shock delivery and/or external rescue/training defibrillator 40a can provide an audio notification of the shock delivery. Note, in a simulated shock delivery in view of an automatic version of external rescue/training defibrillator 40a, the training will automatically progress from the charging pre-shock delivery to the shock delivery, and in a simulated shock delivery in view of a semi-automatic version of external rescue/training defibrillator 40a, the training will a progress from the charging pre-shock delivery to the shock delivery upon a pressing of the shock delivery button of external rescue/training defibrillator 40a.

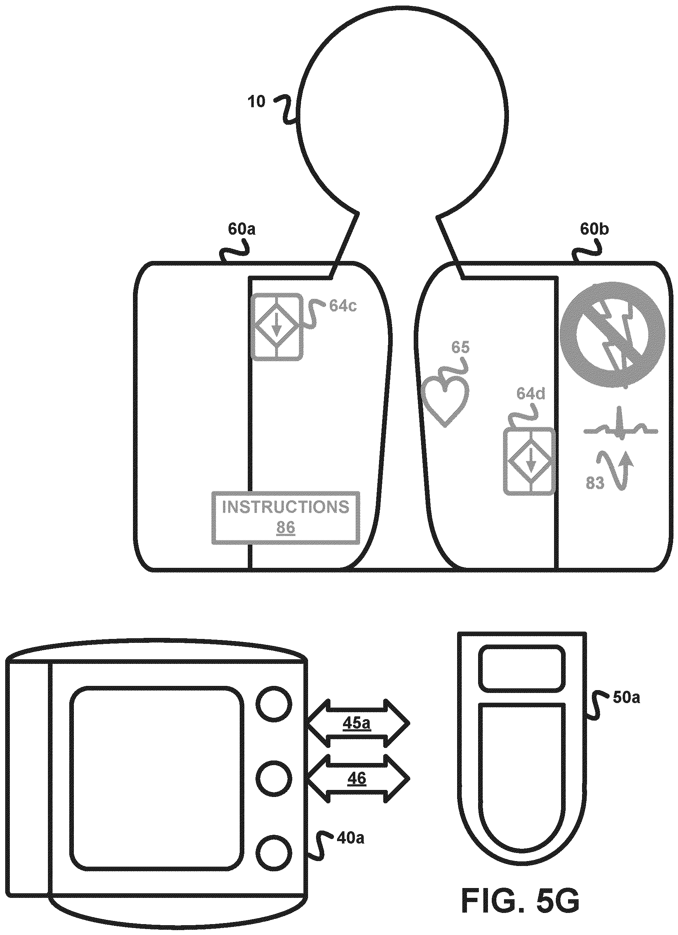

For a simulated no-shock decision in progress without CPR, FIG. 5G illustrates a real-world view through augmented lens 60a and 60b of a CPR manikin 10 without trainee hands 90L and 90R. FIG. 5G further illustrates a screen shoot via augmented lens 60a and 60b of augmented defibrillation electrode pads 64c and 64d highlighting a simulated sensing of electrical cardiac signals of augmented heart 65, and of no-shock decision icon 83. Additionally, instruction box 86 can be provided to inform the trainee of the no-shock decision and/or external rescue/training defibrillator 40a can provide an audio notification of the progress of the no-shock decision.

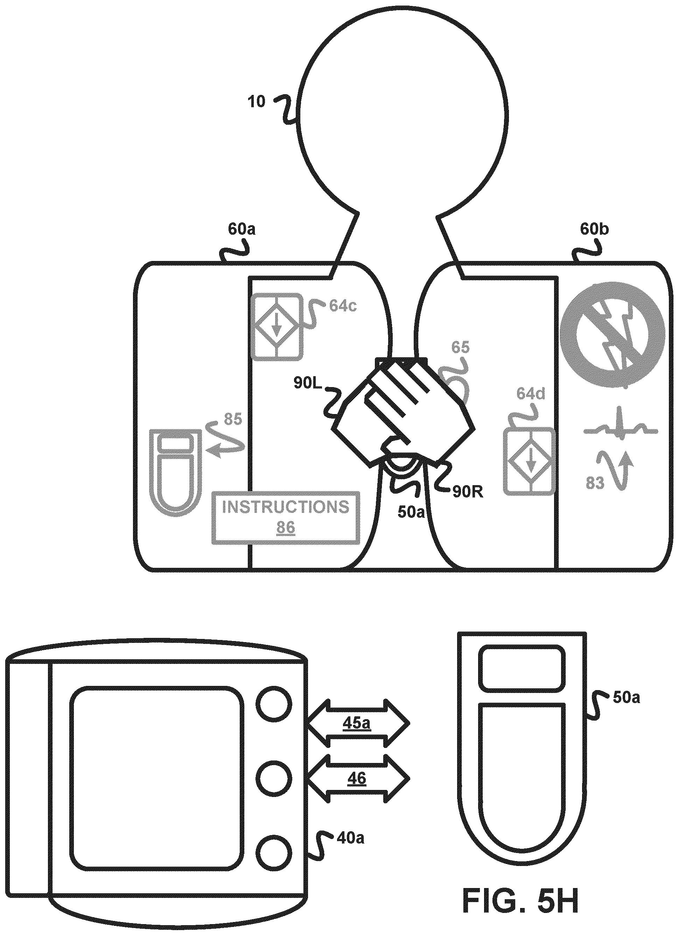

For a simulated no-shock advisory decision with CPR, FIG. 5H illustrates a real-world view through augmented lens 60a and 60b of a CPR manikin 10 with trainee hands 90L and 90R performing CPR via CPR meter 50a per CPR icon 85. FIG. 5H further illustrates a screen shoot via augmented lens 60a and 60b of augmented defibrillation electrode pads 64c and 64d highlighting a sensing of electrical cardiac signals of augmented heart 65, and of no-shock decision icon 83. Additionally, instruction box 86 can be provided to inform the trainee of no-shock decision as well as CPR instructions and/or external rescue/training defibrillator 40a can provide an audio notification of the progress of the no-shock decision as well as CPR instructions.

Referring back to FIG. 3A, in a second exemplary embodiment of stage S110, the controller of the external rescue/training defibrillator executes virtual training applications of the present disclosure (e.g., virtual versions of training applications 44 as shown in FIG. 2 of the present disclosure).

For example, when the training mode of the external rescue/training defibrillator involves a virtual defibrillator trainer of the present disclosure (e.g., virtual defibrillator trainer 70 as shown in FIG. 2 of the present disclosure, the controller of the external rescue/training defibrillator can execute the scenario based training applications including, but not limited to (1) a virtual pad placement scenario, (2) a virtual single shock/single conversion scenario, (3) a virtual multiple shocks/single conversion scenario, (4) a virtual multiple shocks/multiple conversions scenario and (5) a virtual no-shock scenario.

As with the augmented scenarios, FIGS. 4A-4H illustrate exemplary status/feedback icons of the present disclosure that can be utilized in the virtual scenarios.

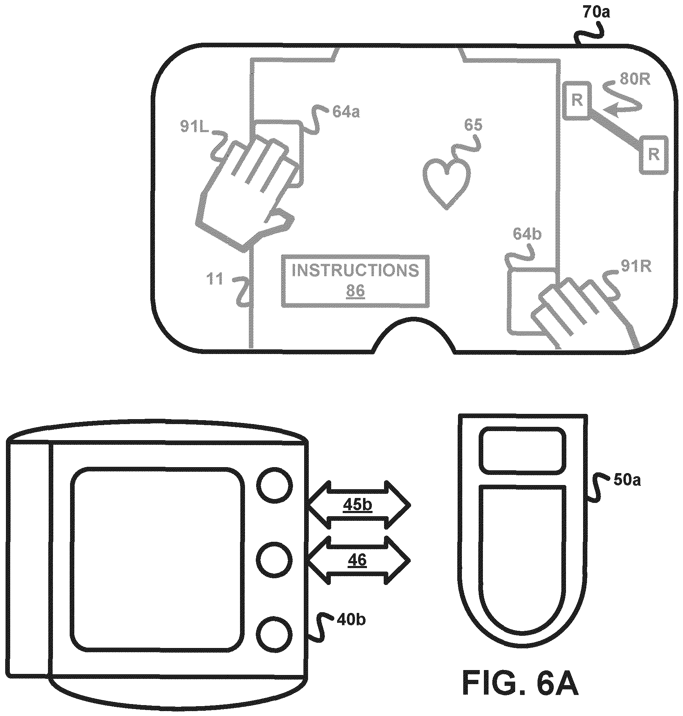

The following description of FIGS. 6A-6H is directed to an exemplary utilization of the status/feedback icon of the present disclosure (e.g., status/feedback icons shown in FIGS. 4A-4H) via exemplary virtual goggles 70a of a virtual defibrillator trainer of the present during an execution of a previously described virtual training scenarios of the present disclosure.

FIGS. 6A-6H further illustrate an exemplary embodiment 40b of external rescue/training defibrillator 40 (FIG. 2 of the present disclosure) having a wireless/wired communication 45b with the virtual defibrillator trainer, and a wireless/wired communication 46 with an exemplary embodiment 50a of CPR meter 50 (FIG. 2 of the present disclosure. While not shown for clarity of the exemplary operation of virtual goggles 70a, a defibrillation rescue/training controller of external rescue/training defibrillator 40b inputs sensing data and/or images from the virtual sensor(s)/camera(s) of the virtual defibrillator trainer to thereby command a virtual graphical processor of the virtual defibrillator trainer to generate the status/feedback icons and instructions as needed during a virtual training scenario.

For these virtual training scenarios, the virtual defibrillator trainer of the present disclosure can generate virtual trainee hands 91L and 91R with or without tracking the trainee hands in the real-world.

For a simulated improper pad placement during a virtual pad placement scenario, FIG. 6A illustrates a virtual training environment through virtual goggles 70a of virtual CPR manikin 11 and simulated trainee hands 91L and 91R. As simulated trainee hands 91L and 91R are being generated the virtual defibrillator trainer, FIG. 6A further illustrates a screen shot via virtual goggles 70a of virtual defibrillation electrode pads 64a and 64b relative to a virtual heart 65, and of a simulated improper pad placement icon 80R informing the simulated trainee hands 91L and 91R have not been properly placed in an anterior-posterior position. Additionally, an instruction box 86 can be provided with instructions for properly placing the electrode pads and/or external rescue/training defibrillator 40b can provide audio instructions for properly placing the electrode pads.

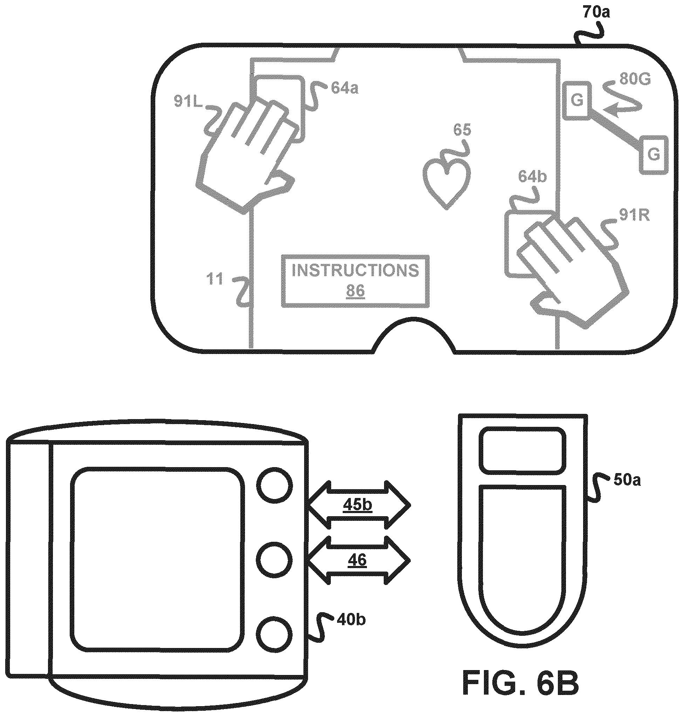

For a simulated proper pad placement during a virtual pad placement scenario, FIG. 6B illustrates a virtual training environment through virtual lens 60a and 60 of a virtual CPR manikin 11 and simulated trainee hands 91L and 91R. As simulated trainee hands 91L and 91R are being generated by the virtual defibrillator trainer, FIG. 6B further illustrates a screen shot via virtual goggles 70a of virtual defibrillation electrode pads 64a and 64b relative to virtual heart 65, and of a simulated proper pad placement icon 80G informing the simulated trainee hands 91L and 91R have been properly placed in an anterior-posterior position. Additionally, an instruction box 86 can be provided with a notification of properly placed electrode pads and/or external rescue/training defibrillator 40b can provide an audio notification s for properly placement of the electrode pads.

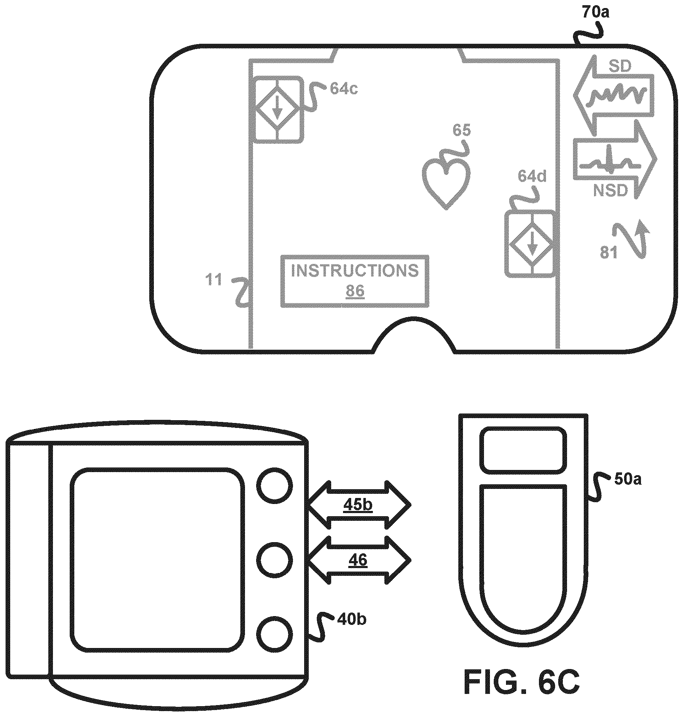

For a simulated shock advisory execution in progress without CPR, FIG. 6C illustrates a virtual training environment through virtual lens 60a and 60 of a virtual CPR manikin 11 without simulated trainee hands 91L and 91R. FIG. 6C further illustrates a screen shoot via virtual lens 60a and 60 of virtual defibrillation electrode pads 64c and 64d highlighting a sensing of electrical cardiac signals of virtual heart 65, and a shock advisory execution in progress icon 81. Additionally, instruction box 86 can be provided to inform the trainee of the progress of the shock advisory execution and/or external rescue/training defibrillator 40b can provide an audio notification of the progress of the shock advisory execution.

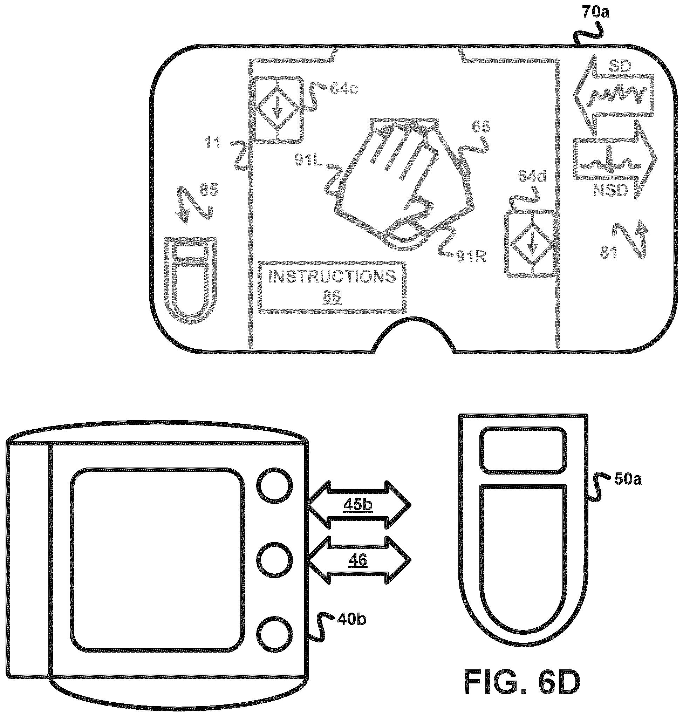

For a simulated shock advisory execution in progress with CPR, FIG. 6D illustrates a virtual training environment through virtual lens 60a and 60 of a virtual CPR manikin 11 with simulated trainee hands 91L and 91R performing CPR via CPR meter 50a per CPR icon 85. FIG. 6D further illustrates a screen shoot via virtual lens 60a and 60 of virtual defibrillation electrode pads 64c and 64d highlighting a sensing of electrical cardiac signals of virtual heart 65, and of shock advisory execution in progress icon 81. Additionally, instruction box 86 can be provided to inform the trainee of the progress of the shock advisory execution as well as CPR instructions and/or external rescue/training defibrillator 40b can provide an audio notification of the progress of the shock advisory execution as well as CPR instructions.

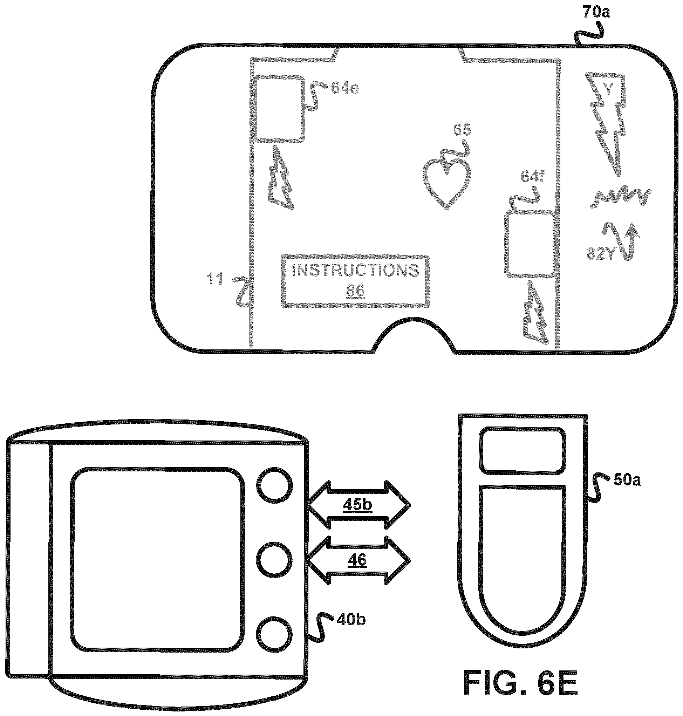

For a simulated shock decision/charging pre-shock delivery, FIG. 6E illustrates a real-world view through virtual goggles 70a of a virtual CPR manikin 11 without virtual trainee hands 91L and 91R. FIG. 6E further illustrates a screen shoot via virtual goggles 70a of virtual defibrillation electrode pads 64e and 64f highlighting a simulated charging pre-shock delivery and of charging pre-shock delivery icon 82Y. Additionally, instruction box 86 can be provided to inform the trainee details of the charging pre-shock delivery and/or external rescue/training defibrillator 40a can provide an audio notification of the charging pre-shock delivery. Also, external rescue/training defibrillator 40a can activate a shock charging button to simulate the charging pre-shock delivery and activate a shock delivery button upon completion of the full charging pre-shock delivery to simulate a semi-automatic version of external rescue/training defibrillator.

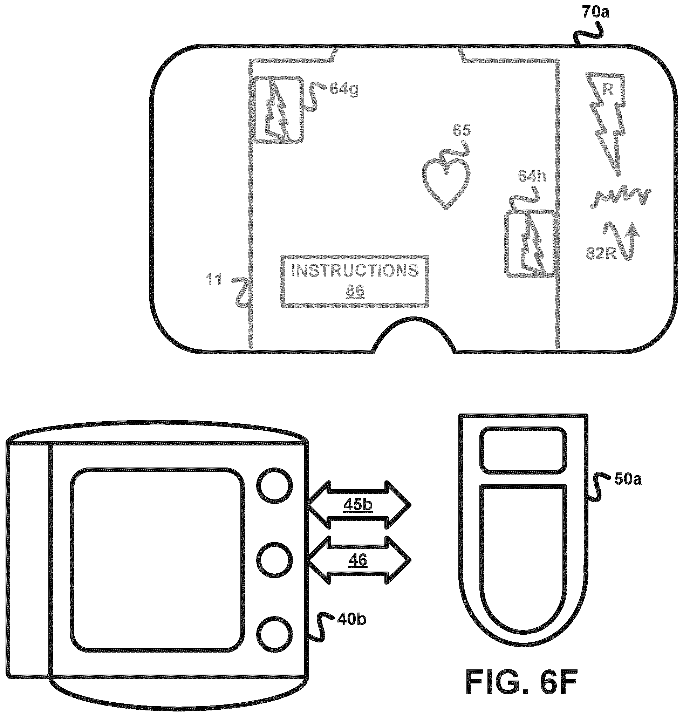

For a simulated shock decision/shock delivery, FIG. 6F illustrates a real-world view through virtual goggles 70a of a virtual CPR manikin 11 without virtual trainee hands 91L and 91R. FIG. 6F further illustrates a screen shoot via virtual goggles 70a of virtual defibrillation electrode pads 64g and 64h highlighting a shock delivery and of a shock delivery icon 82R. Additionally, instruction box 86 can be provided to inform the trainee details of the shock delivery and/or external rescue/training defibrillator 40a can provide an audio notification of the shock delivery. Note, in a simulated shock delivery in view of an automatic version of external rescue/training defibrillator 40a, the training will automatically progress from the charging pre-shock delivery to the shock delivery, and in a simulated shock delivery in view of a semi-automatic version of external rescue/training defibrillator 40a, the training will a progress from the charging pre-shock delivery to the shock delivery upon a pressing of the shock delivery button of external rescue/training defibrillator 40a.

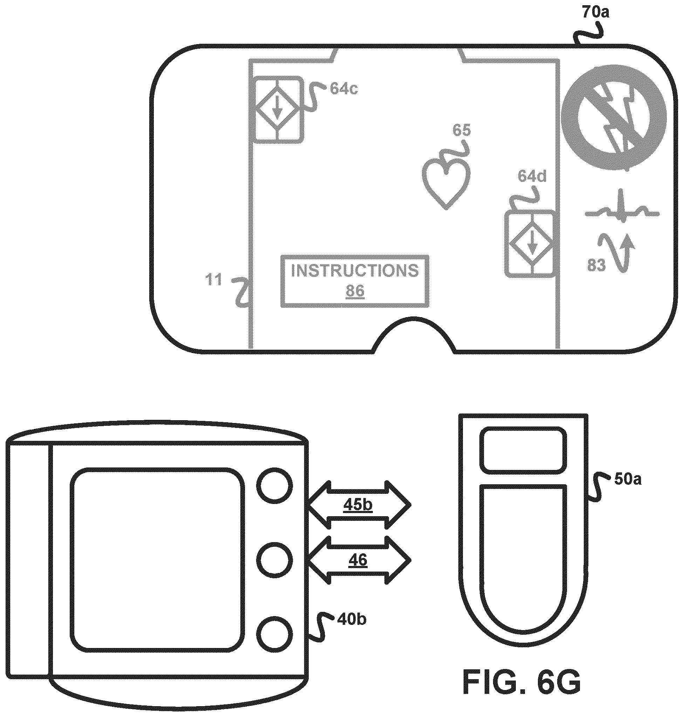

For a simulated no-shock decision in progress without CPR, FIG. 6G illustrates a real-world view through virtual goggles 70a of a virtual CPR manikin 11 without virtual trainee hands 91L and 91R. FIG. 6G further illustrates a screen shoot via virtual goggles 70a of virtual defibrillation electrode pads 64c and 64d highlighting a simulated sensing of electrical cardiac signals of virtual heart 65, and of no-shock decision icon 83. Additionally, instruction box 86 can be provided to inform the trainee of the no-shock decision and/or external rescue/training defibrillator 40a can provide an audio notification of the progress of the no-shock decision.

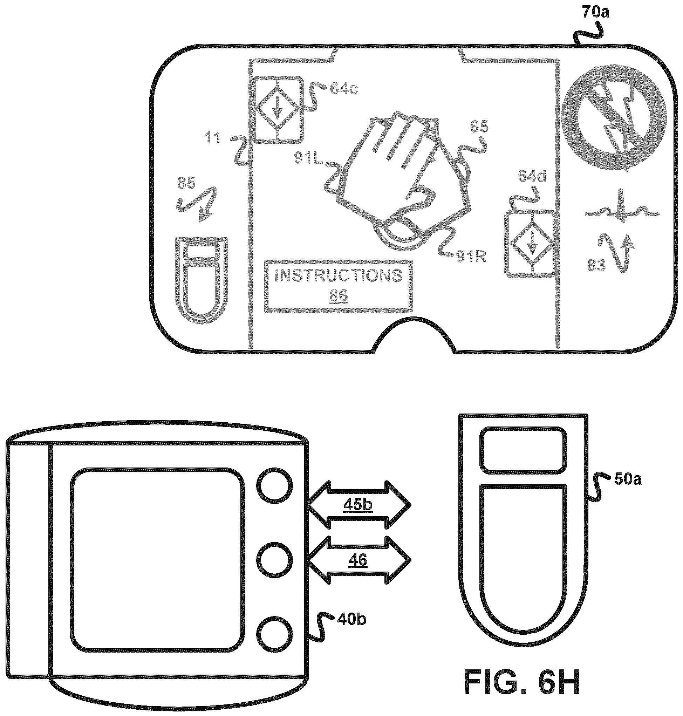

For a simulated no-shock advisory decision with CPR, FIG. 6H illustrates a real-world view through virtual goggles 70a of a virtual CPR manikin 11 with virtual trainee hands 91L and 91R performing CPR via CPR meter 50a per CPR icon 85. FIG. 6H further illustrates a screen shoot via virtual goggles 70a of virtual defibrillation electrode pads 64c and 64d highlighting a sensing of electrical cardiac signals of virtual heart 65, and of no-shock decision icon 83. Additionally, instruction box 86 can be provided to inform the trainee of no-shock decision as well as CPR instructions and/or external rescue/training defibrillator 40a can provide an audio notification of the progress of the no-shock decision as well as CPR instructions.

Referring to FIG. 3B, a flowchart 120 is representative of an exemplary embodiment of a of a training scenario processing method of the present disclosure. A stage S122 of flowchart 120 encompasses an external rescue/training defibrillator of the present disclosure (e.g., external rescue/training defibrillator 40 as shown in FIG. 2 of the present disclosure) executing an ith training scenario. Upon a successful completion of the ith training scenario during a stage S124 of flowchart 120, a stage s126 of flowchart 120 will return to stage S122 to execute the next training scenario during stage S120 or terminate flowchart 120.

In an exemplary practice of flowchart 120, a training supervisor or a trainee powers on an external rescue/training defibrillator the present disclosure using the power button, and press a training selection button to enter training mode. Next, the training supervisor or the trainee connects an augmented defibrillator device of the present disclosure or a virtual defibrillator device of the present disclosure into the external rescue/training defibrillator for training, and presses a flashing training button when prompted to select a training scenario.

The external rescue/training defibrillator will guide the training supervisor or trainee through the training scenario selections whereby the training supervisor or trainee will again press the training selection button to identify a current training scenario and then briefly press an On/Off button of the external rescue/training defibrillator to start the currently selected training scenario.

Upon successful completion of the currently selected training scenario, the training supervisor or trainee can cycle through the scenario selection process again or press and hold the On/Off button of the external rescue/training defibrillator to terminate the training.

Alternative to a scenario selection process, upon pressing the training selection button to enter the training mode, the external rescue/training defibrillator can automatically cycle through the training scenarios until such time the training supervisor or trainee presses and holds the On/Off button of the external rescue/training defibrillator to terminate the training.

For each training scenario, the training supervisor and trainee observe a status indicator of the external rescue/training defibrillator for scenario execution including a red status indication when a training scenario has not been executed successfully and should be repeated, a yellow status indication when a training scenario has been partially executed and should be repeated and a green status indication of a successful execution of the training scenario.

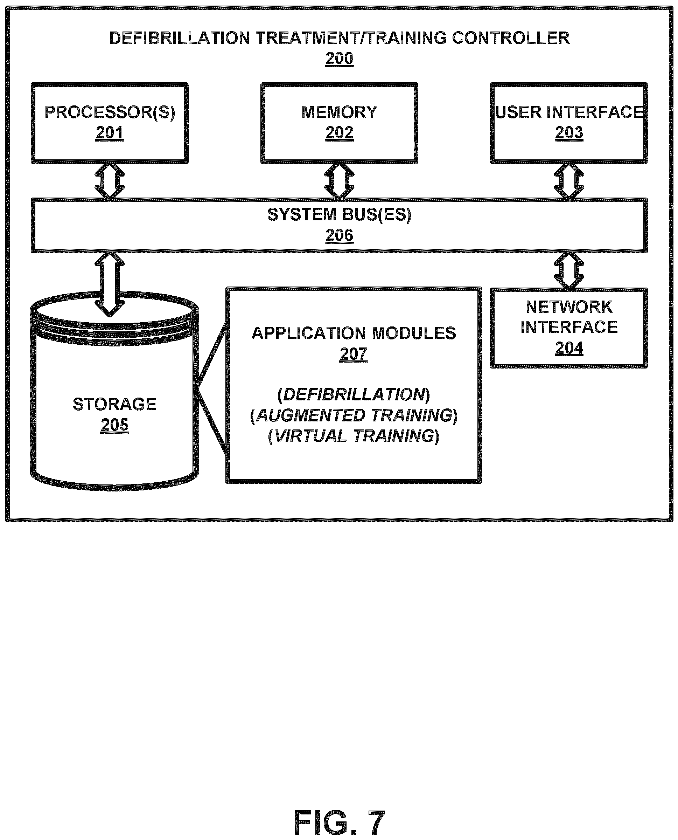

To facilitate a further understanding of the present disclosure, the following description of FIG. 7 describes and teaches exemplary embodiments of a controller in accordance with the present disclosure. From the description of FIG. 7, those having ordinary skill in the art of the present disclosure will appreciate how to apply the present disclosure to make and use additional embodiments of controllers in accordance with the present disclosure.

Referring to FIG. 7 illustrates a defibrillation rescue/training controller 200 as an exemplary embodiment of defibrillation rescue/training controller 41 (FIG. 2). Defibrillation rescue/training controller 200 includes one or more processor(s) 201, memory 202, a user interface 203, a network interface 204, and a storage 205 interconnected via one or more system bus(es) 206.

Each processor 201 can be any hardware device, as known in the art of the present disclosure or hereinafter conceived, capable of executing instructions stored in memory 202 or storage or otherwise processing data. In a non-limiting example, the processor(s) 201 can include a microprocessor, field programmable gate array (FPGA), application-specific integrated circuit (ASIC), or other similar devices.

The memory 202 can include various memories, as known in the art of the present disclosure or hereinafter conceived, including, but not limited to, L1, L2, or L3 cache or system memory. In a non-limiting example, the memory 202 can include static random access memory (SRAM), dynamic RAM (DRAM), flash memory, read only memory (ROM), or other similar memory devices.

In practice, controller 200 also provides control of the user interface (UI) output functions. Specifically, user interface 203 is the primary means for guiding the responder through the protocols of the present disclosure, and so includes at least one of an aural instruction output and a visual display. In particular, user interface 203 may comprise an audio speaker to issue an aural verbal or signal prompt to the responder regarding a state of the rescue, an instruction as to a next step to be taken in the rescue, or regarding instructions responsive to an execution of a particular protocol (e.g., administering CPR and/or delivering a drug). User interface 203 can also convey audible information via a beeper. User interface 203 can also provide visual text or graphical indications on a display. User interface 203 can also convey visual information via a flashing light LED, which may illuminate adjacent graphics or buttons to be pressed. Preferably, controller 200 controls the user interface 201 such that each of these cues is provided in a manner that optimizes the desired response of the responder in the execution of protocols of the present disclosure.

Still referring to FIG. 7, network interface 204 can include one or more devices, as known in the art of the present disclosure or hereinafter conceived, for enabling communication with other components of defibrillator (defibrillator 200 of FIG. 6) or another device, particularly a mechanical CPR device or a CPR coaching device, as known in the art of the present disclosure or hereinafter conceived, in the administration of CPR/chest compression to a patient in accordance with the protocols of the present disclosure and/or in the acquisition of CPR data indicative of the quality of CPR being administered to the patient.

In a non-limiting example, the network interface 204 can include a network interface card (NIC) configured to communicate according to the Ethernet protocol. Additionally, the network interface 414 may implement a TCP/IP stack for communication according to the TCP/IP protocols. Various alternative or additional hardware or configurations for the network interface 204 will be apparent.

The storage 205 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 205 can store instructions for execution by the processor(s) 201 or data upon with the processor(s) 201 may operate. For example, the storage 205 may store a base operating system for controlling various basic operations of the hardware.

The storage 205 can also store an application modules 207 in the form of executable software/firmware for implementing the external defibrillation rescue/training methods of the present disclosure as previously described in the present disclosure.

From the description of FIGS. 2-7 herein, those having ordinary skill in the art will appreciate the numerous benefits of the present disclosure including, but not limited to, (1) visual guidance through an augmented physical learning environment for external rescue/training defibrillator training, (2) interactive and immersive virtual learning environments for external defibrillation training, (3) augmented/virtual scenario-based training with immediate feedback and post-assessment, and (4) portable and accessible solo or collaborative external rescue/training defibrillator training.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which may be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. An external defibrillation rescue/training system, comprising:
at least one of an augmented defibrillator trainer (60) and a virtual defibrillator trainer (70); and
an external rescue/training defibrillator (40) operable between a rescue operation mode for executing a defibrillation treatment for a cardiac emergency and a training operation mode for simulating a defibrillation treatment for the cardiac emergency,
wherein, when the external rescue/training defibrillator (40) is set in the rescue mode, the external rescue/training defibrillator (40) is configured to execute the defibrillation treatment for the cardiac emergency including a conditional shock delivery,
wherein, when the external rescue/training defibrillator (40) is set in the training operation mode and when the external rescue/training defibrillator (40) is in electrical communication with the augmented defibrillator trainer (60), the external rescue/training defibrillator (40) is configured to control a digital simulation of the defibrillation treatment of the cardiac emergency by the augmented defibrillator trainer (60), and
wherein, when the external rescue/training defibrillator (40) is set in the training operation mode and when the external rescue/training defibrillator (40) is in electrical communication with the virtual defibrillator trainer (70), the external rescue/training defibrillator (40) is configured to control a digital simulation of the defibrillation treatment of the cardiac emergency by the virtual defibrillator trainer (70).

2. The external defibrillation rescue/training system of claim 1, wherein, during the digital simulation of the defibrillation treatment by the augmented defibrillator trainer (60), the external rescue/training defibrillator (40) is configured to control a digital simulation by the augmented defibrillator trainer (60) of a defibrillation electrode pad arrangement.

3. The external defibrillation rescue/training system of claim 1, wherein, during the digital simulation of the defibrillation treatment by the virtual defibrillator trainer (70), the external rescue/training defibrillator (40) is configured to control a digital simulation by the virtual defibrillator trainer (70) of a defibrillation electrode pad arrangement.

4. The external defibrillation rescue/training system of claim 1, wherein, during the digital simulation of the defibrillation treatment by the augmented defibrillator trainer (60), the external rescue/training defibrillator (40) is configured to control a digital simulation by the augmented defibrillator trainer (60) of a shock decision derived from a simulated execution of a shock advisory by the external rescue/training defibrillator (40).

5. The external defibrillation rescue/training system of claim 1, wherein, during the digital simulation of the defibrillation treatment by the augmented defibrillator trainer (60), the external rescue/training defibrillator (40) is configured to control a digital simulation by the augmented defibrillator trainer (60) of a no-shock decision derived from a simulated execution of a shock advisory by the external rescue/training defibrillator (40).

6. The external defibrillation rescue/training system of claim 1, wherein, during the digital simulation of the defibrillation treatment by the virtual defibrillator trainer (70), the external rescue/training defibrillator (40) is configured to control a digital simulation by the virtual defibrillator trainer (70) of a shock decision derived from a simulated execution of a shock advisory by the external rescue/training defibrillator (40).

7. The external defibrillation rescue/training system of claim 1, wherein, during the digital simulation of the defibrillation treatment by the virtual defibrillator trainer (70), the external rescue/training defibrillator (40) is configured to control a digital simulation by the virtual defibrillator trainer (70) of a no-shock decision derived from a simulated execution of a shock advisory by the external rescue/training defibrillator (40).

8. An external rescue/training defibrillator (40), comprising:
a defibrillation shock module (44) configured to deliver a defibrillation shock; and
a defibrillation rescue/training controller (41) operable to set the external rescue/training defibrillator (40) in a rescue operation mode for executing a defibrillation treatment for a cardiac emergency or in a training operation mode for simulating the defibrillation treatment for the cardiac emergency,
wherein, when the external rescue/training defibrillator (40) is set in the rescue operation mode, the defibrillation rescue/training controller (41) is configured to control an execution of the defibrillation treatment for the cardiac emergency including a conditional shock delivery by the shock defibrillation module (44),
wherein, when the external rescue/training defibrillator (40) is set in the training operation mode and when the external rescue/training defibrillator (40) is in electrical communication with an augmented defibrillator trainer (60), the defibrillation rescue/training controller (41) is configured to control a digital simulation of the defibrillation treatment of the cardiac emergency by the augmented defibrillator trainer (60) or the virtual defibrillator trainer (70), and
wherein, when the external rescue/training defibrillator (40) is set in the training operation mode and when the external rescue/training defibrillator (40) is in electrical communication with a virtual defibrillator trainer (70), the defibrillation rescue/training controller (41) is configured to control a digital simulation of the defibrillation treatment of the cardiac emergency by the virtual defibrillator trainer (70).

9. The external rescue/training defibrillator (40) of claim 8, wherein, during the digital simulation of the defibrillation treatment by the augmented defibrillator trainer (60), the defibrillator controller (41) is configured to control a digital simulation by the augmented defibrillator trainer (60) of a defibrillation electrode pad arrangement.

10. The external rescue/training defibrillator (40) of claim 8, wherein, during the digital simulation of the defibrillation treatment by the virtual defibrillator trainer (70), the defibrillator controller (41) is configured to control a digital simulation by the virtual defibrillator trainer (70) of a defibrillation electrode pad arrangement.

11. The external rescue/training defibrillator (40) of claim 8, wherein, during the digital simulation of the defibrillation treatment by the augmented defibrillator trainer (60), the defibrillator controller (41) is configured to control a digital simulation by the augmented defibrillator trainer (60) of a shock decision derived from a simulated execution of a shock advisory by the defibrillator controller (41).

12. The external rescue/training defibrillator (40) of claim 8, wherein, during the digital simulation of the defibrillation treatment by the augmented defibrillator trainer (60), the defibrillator controller (41) is configured to control a digital simulation by the augmented defibrillator trainer (60) of a no-shock decision derived from a simulated execution of a shock advisory by the defibrillator controller (41).

13. The external rescue/training defibrillator (40) of claim 8, wherein, during the digital simulation of the defibrillation treatment by the virtual defibrillator trainer (70), the defibrillator controller (41) is configured to control a digital simulation by the virtual defibrillator trainer (70) of a shock decision derived from a simulated execution of a shock advisory by the defibrillator controller (41).

14. The external rescue/training defibrillator (40) of claim 8, wherein, during the digital simulation of the defibrillation treatment by the virtual defibrillator trainer (70), the defibrillator controller (41) is configured to control a digital simulation by the virtual defibrillator trainer (70) of a no-shock decision derived from a simulated execution of a shock advisory by the defibrillator controller (41).

15. An external defibrillation rescue/training method executable by an external rescue/training defibrillator (40) operable between a rescue operation mode for executing a defibrillation treatment for a cardiac emergency and a training operation mode for simulating the defibrillation treatment for the cardiac emergency, the external defibrillation rescue/training method comprising:
executing, by the external rescue/training defibrillator (40) when the external rescue/training defibrillator (40) is set in the rescue operation mode, the defibrillation treatment for the cardiac emergency including a conditional shock delivery;
controlling, by the external rescue/training defibrillator (40) when the external rescue/training defibrillator (40) is set in the training operation mode when the external rescue/training defibrillator (40) is in electrical communication with an augmented defibrillator trainer (60), a digital simulation of the defibrillation treatment of the cardiac emergency by the augmented defibrillator trainer (60) or the virtual defibrillator trainer (70); and
controlling, by the external rescue/training defibrillator (40) when the external rescue/training defibrillator (40) is set in the training operation mode when the external rescue/training defibrillator (40) is in electrical communication with a virtual defibrillator trainer (70), a digital simulation of the defibrillation treatment of the cardiac emergency by the virtual defibrillator trainer (70).
